(19) Europäisches Patentamt
European Patent Office
Office européen des brevets

(11) Numéro de publication : **0 169 139**
**B1**

(12) **FASCICULE DE BREVET EUROPÉEN**

(45) Date de publication du fascicule du brevet : **07.06.89**

(51) Int. Cl.$^4$ : **C 07 D401/04**, A 61 K 31/40, A 61 K 31/495, A 61 K 31/53

(21) Numéro de dépôt : **85401391.9**

(22) Date de dépôt : **09.07.85**

(54) **Nouveaux composés à noyau hétérocyclique azoté, leur préparation et les médicaments qui en contiennent.**

(30) Priorité : **11.07.84 FR 8411039**

(43) Date de publication de la demande : **22.01.86 Bulletin 86/04**

(45) Mention de la délivrance du brevet : **07.06.89 Bulletin 89/23**

(84) Etats contractants désignés : **AT BE CH DE FR GB IT LI LU NL SE**

(56) Documents cités :
**EP-A- 0 088 593**
**EP-A- 0 090 733**
**EP-A- 0 114 770**
**FR-A- 2 539 414**
**Le dossier contient des informations techniques présentées postérieurement au dépôt de la demande et ne figurant pas dans le présent fascicule.**

(73) Titulaire : **SANOFI**
**40, Avenue George V**
**F-75008 Paris (FR)**

(72) Inventeur : **Biziere, Kathleen**
**6, Lotissement Rayons d'Oc**
**F-34170 Clapiers (FR)**
Inventeur : **Chambon, Jean-Pierre**
**Chemin des Truquets Route de Montpellier**
**F-34570 Montarnaud (FR)**
Inventeur : **Hallot, André**
**83, Rue du Juge**
**F-34270 Saint-Gely-Du-Fesc (FR)**

(74) Mandataire : **Gillard, Marie-Louise et al**
**Cabinet Beau de Loménie 55, Rue d'Amsterdam**
**F-75008 Paris (FR)**

**EP 0 169 139 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet de nouveaux composés hétérocycliques présentant d'intéressantes propriétés thérapeutiques à la fois centrales et périphériques.

En particulier, les composés selon l'invention possèdent des activités sur le système nerveux central : activités anticonvulsivante, hypnotique, analgésique, ainsi que des activités sur le système circulatoire et, en particulier, une action antiagrégante plaquettaire.

Une très large famille de dérivés de pyrazine et de triazine-1,2,4, substitués en position 5 et en position 6 par un groupe phényle a été décrite dans la demande de brevet EP-A-00 88 593. Il y est signalé pour l'ensemble de ces composés une activité biochimique sur la liaison aux récepteurs du GABA et des benzodiazépines, et une activité pharmacologique anticonvulsivante, analgésique et anorexigène.

Les composés selon l'invention sont représentés par la formule générale :

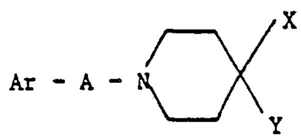

(I)

dans laquelle :

— Ar représente un groupe

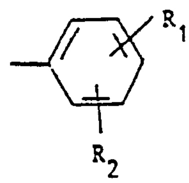

où

$R_1$ représente un atome d'hydrogène, un atome d'halogène, un groupe trifluorométhyle, un groupe alkyle inférieur (1 à 4 atomes de carbone), un groupe alcoxy inférieur (1 à 4 atomes de carbone), un groupe nitro, un groupe cyano ou un groupe hydroxyle ;

$R_2$ représente H ou un atome d'halogène, ou encore

— Ar représente un groupe naphtyle éventuellement substitué par un atome d'halogène ;

— A représente l'un des noyaux hétérocycliques azotés suivants :

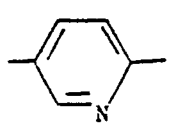 , 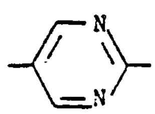 , 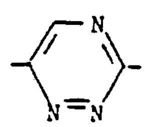 , 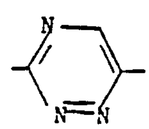

— enfin, considérés isolément, X et Y désignent l'un un atome d'hydrogène et l'autre un groupe $OR_3$ dans lequel $R_3$ représente :

— l'hydrogène ; ou

— un groupe

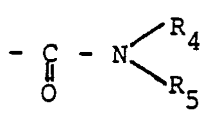

dans lequel $R_4$ désigne l'hydrogène ou un groupe alkyle inférieur (1 à 4 carbones) et $R_5$ désigne un groupe alkyle inférieur (1 à 4 carbones) ou bien $R_4$ et $R_5$ désignent, avec l'atome d'azote auquel ils sont liés, le groupe hydroxy-4 pipéridino ;

— un groupe $COR_5$ dans lequel $R_5$ est tel que ci-dessus ; ou bien X et Y considérés ensemble constituent un groupe oxo (=0).

Plus précisément, les composés selon l'invention répondent à l'une des 4 formules suivantes :

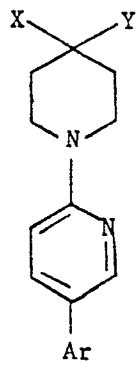

(Ia)

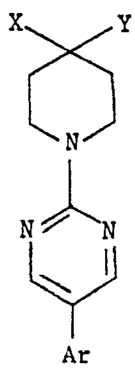

(Ib)

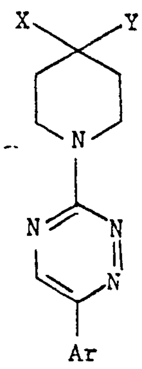

(Ic)

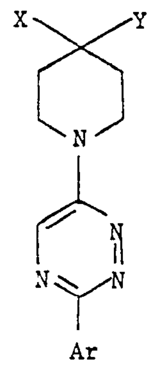

(Id)

dans lesquelles les significations de Ar, X et Y sont telles que définies précédemment.

Les composés sont susceptibles de donner avec les acides minéraux ou organiques des sels d'addition. Ces sels font partie intégrante de l'invention.

D'une façon générale, les composés (1) sont préparés à partir du dérivé halogéné correspondant (le plus souvent le dérivé chloré) sur lequel on fait agir l'amine

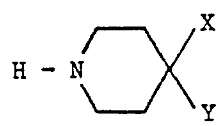

en excès au sein d'un solvant, tel qu'un alcanol (éthanol, propanol, butanol) et en chauffant à une température comprise entre 75 et 120 °C.

Lorsque X et Y représentent ensemble un groupe oxo, il est préférable, pour obtenir de bons résultats, de bloquer ce groupe oxo sous forme d'acétal avant de faire réagir l'amine sur le dérivé halogéné. Après condensation de l'amine, comme indiqué ci-dessus, on libère le groupe oxo par traitement en milieu acide.

Lorsque X = H et Y représente un groupe

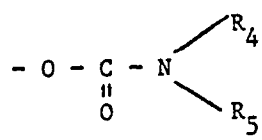

les composés (I) peuvent s'obtenir à partir des composés correspondants où X = H, Y = OH.

Dans un premier temps, on prépare l'ester activé correspondant par action d'un chloroformiate, tel que le chloroformiate de phényle, selon un procédé connu :

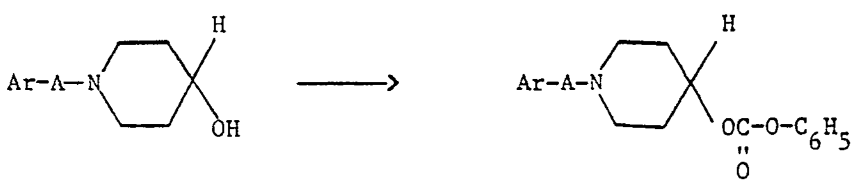

Ce dernier, chauffé avec l'amine

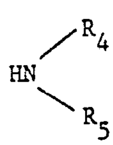

en solution dans un solvant inerte, conduit au composé (I) où X = H et Y =

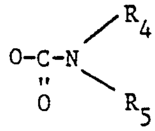

Les composés où X est H et Y est un groupe O—CO—$R_5$ peuvent être préparés par acylation selon un procédé connu des composés correspondants où X = H et Y = OH.

Enfin, lorsque le groupe Ar représente un hydroxyphényle, les composés (I) peuvent être préparés par déméthylation des composés (I) correspondants où Ar représente un groupe méthoxyphényle.

Les dérivés halogénés utilisés pour la préparation des composés selon l'invention sont connus ou peuvent être préparés selon des procédés connus.

Dérivés de la pyridine

Les dérivés chlorés peuvent être préparés comme indiqué dans le brevet européen EP-B-00 17 438 selon le schéma réactionnel suivant :

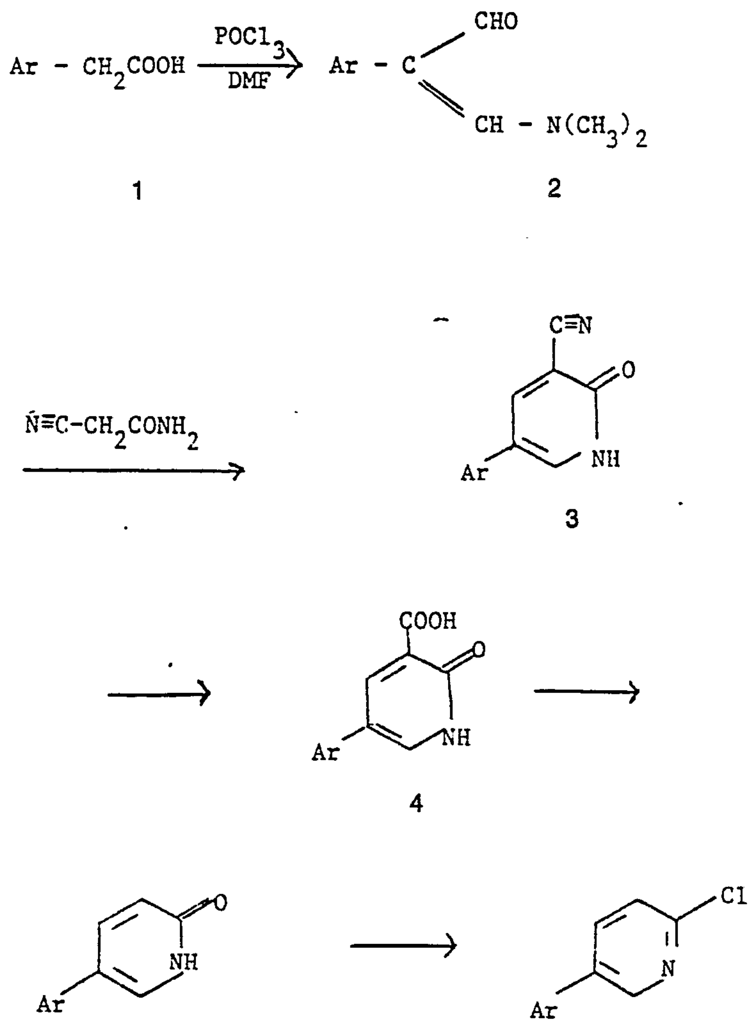


1 2 3 4 5 6

Le traitement d'un acide arylacétique 1 par l'oxychlorure de phosphore et le diméthylformamide conduit au dérivé du propène-2-al 2 qui, condensé avec le cyanacétamide, conduit au composé cyclique 3. Celui-ci, par hydrolyse du nitrile, conduit au composé 4 qui est décarboxylé pour fournir le composé 5.

Enfin, l'action de l'oxychlorure de phosphore conduit au dérivé halogéné 6 attendu.

Dérivés de la pyrimidine

Selon le brevet des Etats-Unis d'Amérique n° 4 209 621, on peut préparer les dérivés chlorés de la pyrimidine suivant le schéma réactionnel suivant :

(Voir schéma page 5)

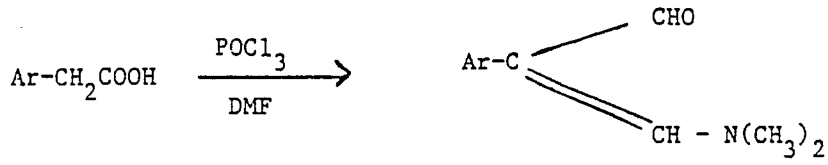


1 2

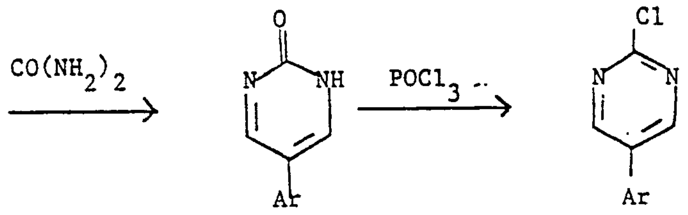


7 8

La première étape, comme indiqué précédemment, conduit au propène-2-al 2. Celui-ci, par action de l'urée, fournit la pyrimidinone 7 qui, traitée par $POCl_3$, conduit au dérivé chloré désiré.

Dérivés des triazines-1,2,4

a) Dérivés chlorés en 3

Selon la méthode décrite dans Journal of Heterocyclic Chemistry 16, 1392-1407 (1979), on peut obtenir ces composés selon le schéma réactionnel :

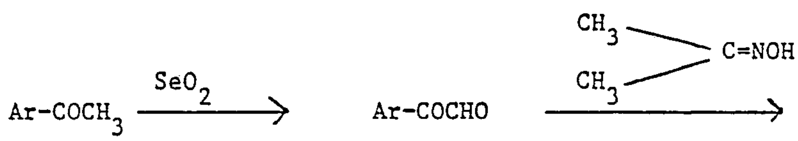


9 10

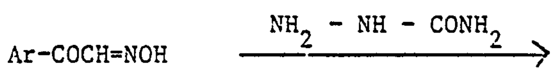


11

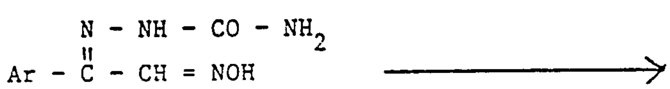

12

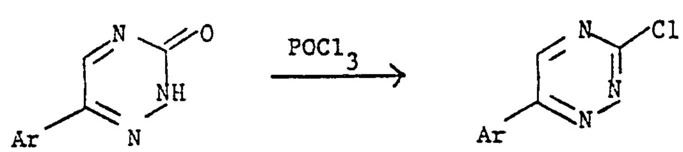


13 14

L'oxydation par le dioxyde de sélénium d'une acétophénone 9 conduit au phénylglyoxal 10 qui conduit à l'oxime correspondante 11 par une méthode d'échange d'oxime. Cette dernière est transformée en semicarbazone 12 que l'on cyclise en 13 par chauffage en milieu acide dilué.

Par action de l'oxychlorure de phosphore, on obtient le dérivé chloré 14.

b) Dérivés chlorés en 6

Pour obtenir de tels dérivés, on peut s'inspirer de la technique décrite dans la demande de brevet européen EP-A-0080296.

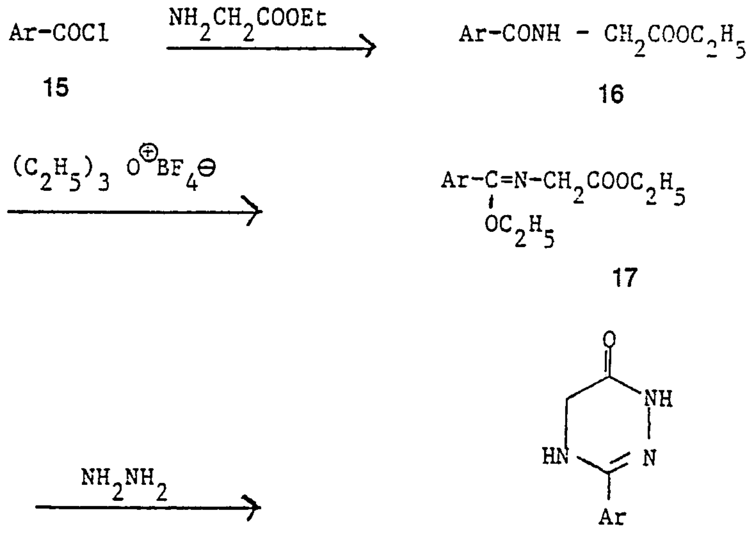


15 16 17 18

Sur le chlorure d'acide 15, l'action du chlorhydrate de glycinate d'éthyle conduit au dérivé de l'acide hippurique 16. Celui-ci traité par le fluoroborate de triéthyloxonium conduit à 17 que l'on cyclise avec de l'hydrazine pour obtenir la tétrahydrotriazine-1,2,4 one-6,18.

A partir de ce composé, l'action du brome dans l'acide acétique fournit le dérivé dihydro correspondant, puis l'action de l'oxychlorure de phosphore conduit au dérivé chloré :

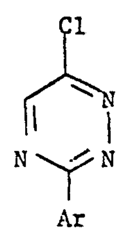

19

Les exemples suivants illustrent l'invention sans en limiter la portée.

Exemple 1

(Chloro-2 phényl)-5 (hydroxy-4 pipéridino)-2 pyridine, maléate acide (SR 42482 A)

a) (Chloro-2 phényl)-2 diméthylamino-3 propène-2-al

73 g de diméthylformamide sont refroidis à — 50 °C. Ajouter 92 g d'oxychlorure de phosphore goutte à goute, puis 34,2 g d'acide chloro-2 phénylacétique et agiter le mélange réactionnel à température ambiante pendant 30 min. Chauffer ensuite à 70-80 °C pendant 5 h 30 min. Alcaliniser le mélange réactionnel avec du carbonate de potassium, ajouter 200 ml de toluène et chauffer au bain-marie pendant 1 h.

Décanter la phase organique, la laver à l'eau, décanter, sécher sur sulfate de sodium, filtrer et concentrer sous vide. Recristalliser le résidu dans le mélange éther isopropylique/chlorure de méthylène.

Poids : 5,4 g ; F. 86-88 °C.

b) (Chloro-2 phényl)-5 dihydro-1,2 oxo-2 pyridinecarbonitrile-3

3,3 g de sodium sont dissous dans 150 ml de méthanol. Ajouter à la solution 6,1 g de cyanoacétamide, puis 5,4 g du produit obtenu précédemment et chauffer le mélange réactionnel à reflux pendant 16 h.

Séparer un insoluble par filtration, ajouter 10 ml d'acide acétique et 500 ml d'eau aux eaux-mères, filtrer le précipité et le recristalliser dans l'éthanol absolu.

Poids : 3,2 g ; F. 254-256 °C

c) Acide (chloro-2 phényl)-5 dihydro-1,2 oxo-2 pyridinecarboxylique-3

12,2 g du produit obtenu précédemment sont chauffés à reflux dans un mélange de 100 ml d'acide chlorhydrique et 100 ml d'acide acétique pendant 16 h.

Refroidir le mélange réactionnel, filtrer le précipité, le rincer à l'eau et le sécher sous vide.

Poids : 12,4 g ; F. > 260 °C

d) (Chloro-2 phényl)-5 dihydro-1,2 oxo-2 pyridine

12,4 g du produit obtenu précédemment dans 80 ml de quinoléine et 1,2 g de cuivre réduit sont chauffés à reflux sous agitation pendant 4 h.

Verser le mélange réactionnel dans 1,5 l d'une solution d'acide chlorhydrique à 20 %, filtrer le précipité, le rincer à l'eau, le sécher sous vide et chromatographier sur gel de silice ; éluant : acétate d'éthyle, puis acétate d'éthyle/méthanol 90/10.

Eliminer trois impuretés de tête et concentrer la fraction suivante sous vide :

Poids : 4,3 g ; F. 156-158 °C.

e) Chloro-2 (chloro-2 phényl)-5 pyridine

4,3 g du produit obtenu précédemment sont chauffés à reflux dans 100 ml d'oxychlorure de phosphore en présence de 0,5 ml de diméthylformamide pendant 4 jours.

Concentrer l'excès d'oxychlorure de phosphore sous vide, reprendre le résidu dans une solution de carbonate de sodium, extraire au chlorure de méthylène, décanter, sécher sur sulfate de sodium, filtrer et concentrer sous vide.

Chromatographier le résidu sur gel de silice ; éluant : acétate d'éthyle. Concentrer la première fraction sous vide.

Poids : 3 g ; F. 100-102 °C.

f) SR 42 482 A

3 g du produit obtenu précédemment et 3,9 g d'hydroxy-4 pipéridine sont chauffés à reflux dans 100 ml de butanol pendant 48 h.

Concentrer le butanol sous vide, reprendre le résidu dans l'eau, extraire à l'acétate d'éthyle, décanter la phase organique, sécher sur sulfate de sodium, filtrer et concentrer sous vide. Chromatographier le résidu sur gel de silice ; éluant : acétate d'éthyle.

Eliminer trois impuretés de tête et concentrer la fraction suivante sous vide. Reprendre le résidu dans 100 ml d'acétate d'éthyle, ajouter 0,6 g d'acide maléique, chauffer à ébullition, filtrer et laisser cristalliser le maléate.

Poids : 1,6 g ; F. 140-142 °C

## Exemple 2

(Dichloro-2,4 phényl-5 (hydroxy-4 pipéridino)-2 pyridine (SR 42903)

En opérant comme dans l'exemple 1, mais en remplaçant l'acide chloro-2 phénylacétique par une quantité équivalente d'acide dichloro-2,4 phénylacétique, on obtient successivement :

— le (dichloro-2,4 phényl)-2 diméthylamino-3 propène-2 al ;
F. 112-114 °C (acétate d'éthyle)
— le (dichloro-2,4 phényl)-5 dihydro-1,2 oxo-2 pyridinecarbonitrile-3 ; F. > 260 °C (éthanol)
— l'acide (dichloro-2,4 phényl)-5 dihydro-1,2 oxo-2 pyridine carboxylique-3 ; F. > 260 °C
— la (dichloro-2,4 phényl)-5 dihydro-1,2 oxo-2 pyridine ; F. 170-172 °C (chromatographié)
— le chloro-2 (dichloro-2,4 phényl)-5 pyridine ; F. 150 °C
— SR 42 903 isolé sous forme de base ; F. 132-134 °C (éther isopropylique)

## Exemple 3

(Chloro-2 phényl)-5 (oxo-4 pipéridino)-2 pyridine, maléate acide (SR 42696 A)

a) (Chloro-2 phényl)-5 (dioxo-1,4 aza-8 spiro [4,5] décyl-8)-2 pyridine

5 g de chloro-2 (chloro-2 phényl)-5 pyridine (exemple 1 e) et 9,58 g de dioxa-1,4 aza-8 spiro [4,5] décane sont chauffés à reflux dans 100 ml de butanol pendant 6 jours.

Concentrer le butanol sous vide, reprendre le résidu dans l'eau additionnée d'acide chlorhydrique, laver à l'éther, alcaliniser la phase aqueuse avec une solution de carbonate de sodium, extraire à l'acétate d'éthyle, décanter la phase organique, sécher sur sulfate de sodium, filtrer et concentrer sous vide.

Poids : 3,1 g (huile brune)

b) SR 42 696 A

3,1 g du produit obtenu précédemment sont chauffés à reflux dans une solution de 60 ml d'eau et 40 ml d'acide formique pendant 3 h.

Verser le mélange réactionnel dans une solution glacée de soude, extraire au chlorure de méthylène, décanter, sécher sur sulfate de sodium, filtrer et concentrer sous vide.

Chromatographier le résidu sur gel de silice ; éluant : chloroforme/méthanol 95/5.

Eliminer les têtes puis concentrer la deuxième fraction sous vide et faire cristalliser le maléate dans l'acétate d'éthyle.

Poids : 1,3 g ; F. 146-148 °C.

## Exemple 4

(Chloro-2 phényl)-5 (hydroxy-4 pipéridino)-2 pyrimidine (SR 42 505)

a) (Chloro-2 phényl)-5 dihydro-1,2 oxo-2 pyrimidine

10 g de (chloro-2 phényl)-2 diméthylamino-3 propène-2-al (exemple 1 a) et 6 g d'urée sont chauffés à reflux dans 40 ml d'éthanol en présence de 10 ml d'acide chlorhydrique pendant 4 h.

Concentrer l'éthanol sous vide, reprendre le résidu dans l'eau, alcaliniser à la soude, filtrer le précipité, le rincer à l'éthanol, puis à l'éther.

Poids : 11 g ; F. > 260 °C

b) Chloro-2 (chloro-2 phényl)-5 pyrimidine

11 g de produit obtenu précédemment sont chauffés à reflux dans 100 ml d'oxychlorure de phosphore pendant 2 h.

Concentrer l'excès d'oxychlorure de phosphore sous vide, reprendre le résidu dans l'eau additionnée de carbonate de sodium, extraire à l'acétate d'éthyle, décanter, sécher sur sulfate de sodium, filtrer et concentrer.

Poids : 3,2 g ; F. 152-154 °C

c) SR 42 505

3,2 g du produit obtenu précédemment et 4,3 g d'hydroxy-4 pipéridine sont chauffés à reflux dans 100 ml d'éthanol pendant 3 h.

Concentrer l'éthanol sous vide, reprendre le résidu dans l'eau additionnée de carbonate de sodium, extraire à l'acétate d'éthyle, décanter, sécher sur sulfate de sodium, filtrer et concentrer sous vide.

Chromatographier le résidu sur gel de silice ; éluant : acétate d'éthyle. Eliminer une impureté de tête et concentrer la fraction suivante sous vide. Recristalliser le résidu dans l'éther isopropylique/chlorure de méthylène.

Poids : 3,2 g ; F. 122 °C.

## Exemples 5 à 9

En opérant comme dans l'exemple 4, mais en faisant varier l'acide arylacétique de départ, on obtient de même successivement :

— la (dichloro-2,4 phényl)-5 dihydro-1,2 oxo-2 pyrimidine ; F. > 260 °C,
— la (chloro-4 phényl)-5 dihydro-1,2 oxo-2 pyrimidine ; F. 140 °C environ,
— la (chloro-3 phényl)-5 dihydro-1,2 oxo-2 pyrimidine ; F. 250-252 °C (décomposition),
— la (méthoxy-4 phényl)-5 dihydro-1,2 oxo-2 pyrimidine ; F. 258-260 °C,
— la (naphtyl-1)-5 dihydro-1,2 oxo-2 pyrimidine ; F. 260 °C, puis
— la chloro-2 (dichloro-2,4 phényl)-5 pyrimidine ; F. 176-178 °C,
— la chloro-2 (chloro-4 phényl)-5 pyrimidine,
— la chloro-2 (chloro-3 phényl)-5 pyrimidine ; F. 124-126 °C (chromatographie),
— la chloro-2 (méthoxy-4 phényl)-5 pyrimidine ; F. 132-134 °C,
— la chloro-2 (naphtyl-1)-5 pyrimidine chromatographiée sur gel de silice (éluant chlorure de méthylène),

et enfin :

— la (dichloro-2,4 phényl)-5 (hydroxy-4 pipéridino)-2 pyrimidine (SR 42 824) ; F. 122-124 °C (éther isopropylique),

— la (chloro-4 phényl)-5 (hydroxy-4 pipéridino)-2 pyrimidine (SR 42 921) ; F. 168-170 °C (acétate d'éthyle),

— la (chloro-3 phényl)-5 (hydroxy-4 pipéridino)-2 pyrimidine (SR 42 922) ; F. 160-162 °C (acétate d'éthyle),

— la (méthoxy-4 phényl)-5 (hydroxy-4 pipéridino)-2 pyrimidine (SR 43 159) ; F. 158-160 °C (éthanol),

— la (naphtyl-1)-5 (hydroxy-4 pipéridino)-2 pyrimidine (SR 43 139) ; F. 130-132 °C (éther isopropylique).

Exemple 10

(Acétoxy-4 pipéridino)-2 (dichloro-2,4 phényl)-5 pyrimidine (SR 43 651)

On chauffe au reflux pendant 4 h 6 g d'(hydroxy-4 pipéridino)-2 (dichloro-2,4 phényl)-5 pyrimidine (exemple 5) et 60 ml d'anhydride acétique.

On évapore à siccité sous vide et reprend le résidu dans une solution aqueuse de carbonate de sodium à 10 %. On extrait avec du chlorure de méthylène, sèche la solution sur sulfate de sodium et concentre à siccité sous vide. On chromatographie le résidu sur gel de silice. En éluant au chlorure de méthylène, on obtient le produit attendu.

Après recristallisation dans l'éther isopropylique, F. 100-102 °C ; poids : 1,1 g.

Exemple 11

(Méthylcarbamoyloxy-4 pipéridino)-2 (chloro-2 phényl)-5 pyrimidine (SR 43 543)

a) (Phénoxycarbonyloxy-4 pipéridino)-2 (chloro-2 phényl)-5 pyrimidine

A la solution de 12,7 g de (chloro-2 phényl)-5 (hydroxy-4 pipéridino-2 pyrimidine (exemple 4) dans 50 ml de pyridine refroidie à 5 °C, on ajoute lentement 11,4 ml de chloroformiate de phényle. Après la fin de l'addition, on chauffe à 60 °C pendant 3 h.

On évapore la pyridine sous vide. On reprend le résidu dans la soude diluée et extrait avec du chlorure de méthylène. On sèche la solution sur sulfate de sodium puis évapore le solvant sous vide.

On chromatographie le résidu sur colonne de gel de silice. En éluant avec de l'acétate d'éthyle, on obtient le produit attendu. On cristallise dans l'acétate d'éthyle ; F. 114-116 °C ; poids : 11,5 g.

b) SR 43 543

On chauffe au reflux pendant 18 h le mélange de 4,1 g du produit obtenu ci-dessus et 3 ml d'une solution aqueuse à 40 % de méthylamine dans 80 ml d'acétone. On évapore l'acétone sous vide et reprend le résidu dans une solution aqueuse diluée d'acide chlorhydrique. On extrait avec de l'acétate d'éthyle et sépare la phase aqueuse qu'on alcalinise avec de l'ammoniaque. On extrait avec de l'acétate d'éthyle et sèche la solution sur sulfate de sodium. On évapore le solvant sous vide et cristallise le résidu dans le pentane. On recristallise dans l'éther pour obtenir 1,1 g du produit attendu ; F. 104-106 °C.

Exemples 12 et 13

En opérant comme dans l'exemple 11b), à partir de l'ester activité préparé en a), mais en faisant varier l'amine, on obtient :

— la (diméthylcarbamoyloxy-4 pipéridino)-2 (chloro-2 phényl)-5 pyrimidine (SR 43 537), F. 86-88 (éther isopropylique),

— l'[(hydroxy-4 pipéridino carbonyloxy)-4 pipéridino]-2 (chloro-2 phényl)-5 pyrimidine (SR 43 516), F. 124-126 °C (acétate d'éthyle).

Exemple 14

(Diméthylcarbamoyloxy-4 pipéridino)-2 (dichloro-2,4 phényl)-5 pyrimidine (SR 43 539)

a) (Phénoxycarbonyloxy-4 pipéridino)-2 (dichloro-2,4 phényl)-5 pyrimidine

Préparé selon l'exemple 11a) à partir du composé hydroxylé de l'exemple 5.
F. 168-170 °C (acétate d'éthyle).

b) SR 43 539

Préparé comme dans l'exemple 11b) à partir du composé préparé ci-dessus et de diméthylamine.
F. 122-124 °C (hexane).

Exemple 15

(Chloro-2 phényl)-6 (hydroxy-4 pipéridino)-3 triazine-1,2,4 (SR 42 524)

7,4 g de chloro-3 (chloro-2 phényl)-6 triazine-1,2,4 préparée selon J. Het. Chem. 16, 1402 (1979) et 9 g d'hydroxy-4 pipéridine sont chauffés à reflux dans 150 ml d'éthanol pendant 3 h.

Concentrer l'éthanol sous vide, reprendre le résidu dans l'eau, alcaliniser avec une solution de carbonate de sodium à 10 %, extraire à l'acétate d'éthyle, décanter la phase organique, sécher sur sulfate de sodium, filtrer et concentrer sous vide.

Recristalliser le résidu dans l'acétate d'éthyle.

Poids 5,4 g ; F. 162-164 °C.

Exemple 16

(Dichloro-2,4 phényl)-6 (hydroxy-4 pipéridino)-3 triazine-1,2,4 (SR 42 825)

a) (Dichloro-2,4 phényl)-glyoxaldoxime

36,4 g d'oxyde de sélénium sont mis en solution dans 245 ml de dioxanne en présence de 7,3 ml d'eau. Ajouter 60,4 g de dichloro-2,4 acétophénone et chauffer le mélange réactionnel à reflux sous agitation pendant 18 h. Filtrer la solution, ajouter 100 ml d'eau et ajuster à pH-4-5 avec une solution de soude à 5 %.

Ajouter alors 24,6 g d'acétoxime et agiter le mélange réactionnel à température ambiante pendant 8 jours.

Filtrer une deuxième fois, concentrer le dioxanne sous vide, reprendre le résidu dans l'eau, extraire à l'éther, décanter, sécher sur sulfate de sodium, filtrer et concentrer sous vide.

Poids : 62,2 g, huile jaune.

b) Semicarbazone de (dichloro-2,4 phényl) glyoxaldoxime

62 g du produit obtenu précédemment, 31,8 g de chlorhydrate de semicarbazide et 38,9 g d'acétate de sodium trihydraté sont chauffés à 50 °C dans 350 ml d'éthanol à 50 %, sous agitation, pendant 2 h 30 min.

Refroidir le mélange réactionnel et filtrer le précipité.

Poids : 75 g

c) (Dichloro-2,4 phényl)-6 dihydro-2,3 oxo-3 triazine-1,2,4

75 g du produit obtenu précédemment sont chauffés à reflux dans 2 litres d'acide chlorhydrique à 5 % pendant 1 h.

Filtrer le précipité et le chauffer à reflux dans 350 ml d'acide acétique pendant 18 h. Concentrer l'acide acétique sous vide, reprendre le résidu dans l'eau, filtrer le précipité, le reprendre dans l'éthanol et sécher sous vide.

Poids : 36 g ; F. environ 160 °C.

d) (Chloro-3 (dichloro-2,4 phényl)-6 triazine-1,2,4

18 g du produit obtenu précédemment sont chauffés à reflux dans 180 ml d'oxychlorure de phosphore en présence de 2 ml de diméthylformamide pendant 3 h 30 min.

Concentrer l'excès d'oxychlorure de phosphore sous vide, reprendre le résidu dans l'eau, extraire à l'acétate d'éthyle, décanter, sécher sur sulfate de sodium, filtrer et concentrer sous vide.

Poids : 15,7 g

e) SR 42 825

7,8 g du produit obtenu précédemment et 9,1 g d'hydroxy-4 pipéridine sont chauffés à reflux dans 150 ml d'éthanol absolu pendant 6 h.

Concentrer l'éthanol sous vide, reprendre le résidu dans l'eau, alcaliniser avec une solution de carbonate de sodium, extraire à l'acétate d'éthyle, décanter, sécher sur sulfate de sodium, filtrer et concentrer sous vide.

Chromatographier le résidu sur gel de silice ; éluant : acétate d'éthyle. Eliminer les impuretés de tête et concentrer la fraction suivante sous vide. Recristalliser le résidu de l'acétate d'éthyle.

Poids : 1,4 g ; F. 128-130 °C.

Exemple 17

(Chloro-2 phényl)-6 (oxo-4 pipéridino)-3 triazine-1,2,4 (SR 42 642)

1,5 g de chloro-3 (chloro-2 phényl)-6 triazine-1,2,4, 3,05 g de pipéridone-4 monohydrate chlorhydrate et 3,1 g de carbonate de sodium sont chauffés à reflux dans 100 ml d'éthanol pendant 4 h.

Concentrer l'éthanol sous vide, reprendre le résidu dans l'eau, extraire à l'acétate d'éthyle, décanter, sécher sur sulfate de sodium, filtrer et concentrer sous vide. Chromatographier le résidu sur gel de silice ; éluant : acétate d'éthyle.

Concentrer la première fraction sous vide. Recristalliser le résidu de l'éther isopropylique/chlorure de méthylène.

Poids : 0,9 g ; F. 170-172 °C.

Exemple 18

(Dichloro-2,4 phényl)-6 (oxo-4 pipéridino)-3 triazine-1,2,4 (SR 42 826)

On opère comme dans l'exemple 17, à partir du dérivé chloré obtenu à l'exemple 16d). De la même façon, on isole le produit attendu.

F. 120-122 °C (éther isopropylique-chlorure de méthylène).

Exemple 19

(Chloro-4 phényl)-3 (hydroxy-4 pipéridino)-6 triazine-1,2,4 (SR 42 833)

a) N-(chloro-4 benzoyl)-aminoacétate d'éthyle

30 g de chlorure d'acide chloro-4 benzoyle et 50,2 g de chlorhydrate de glycinate d'éthyle sont chauffés à reflux sous agitation dans 150 ml de benzène pendant 8 h.

Verser le mélange réactionnel dans une solution diluée de carbonate de sodium, extraire à l'acétate d'éthyle, décanter le phase organique, sécher sur sulfate de sodium, filtrer et concentrer sous vide. Reprendre le résidu dans l'éther isopropylique et filtrer le précipité.

Poids : 60,5 g ; F. 118 °C.

b) N-(éthoxycarbonylméthyl) chloro-4 benzimidate d'éthyle

44 g du produit obtenu précédemment et 50,7 g de triéthyloxonium tétrafluoroborate sont mis en solution dans 250 ml de chlorure de méthylène et agités à température ambiante pendant 6 jours. Ajouter goutte à goutte sous agitation 37,6 g de carbonate de potassium en solution dans 70 ml d'eau, diluer avec 200 ml de chlorure de méthylène, décanter la phase organique, sécher sur sulfate de sodium, filtrer et concentrer sous vide.

Reprendre le résidu dans l'éther de pétrole, filtrer un insoluble et concentrer les eaux-mères sous vide.

Poids : 35,4 g, huile jaune.

c) (Chloro-4 phényl)-3 tétrahydro-1,4,5,6 oxo-6 triazine-1,2,4

35,4 g du produit obtenu précédemment et 12,75 ml d'hydrate d'hydrazine sont chauffés à reflux dans 300 ml d'éthanol absolu pendant 2 h.

Laisser refroidir le mélange réactionnel, filtrer le précipité et le recristalliser dans du méthanol.

Poids : 14,4 g ; F. > 260 °C.

d) (Chloro-4 phényl)-3 dihydro-1,6 oxo-6 triazine-1,2,4

3 g du produit obtenu précédemment sont mis en suspension dans 30 ml d'acide acétique et chauffés sous agitation à 40 °C. Ajouter goutte à goutte à cette température 0,8 ml de brome en solution dans 10 ml d'acide acétique et chauffer ensuite le mélange réactionnel à reflux pendant une demi-heure.

Concentrer l'acide acétique sous vide, reprendre le résidu dans l'eau, extraire à l'acétate d'éthyle, laver avec une solution d'acide chlorhydrique diluée, décanter, sécher sur sulfate de sodium, filtrer et concentrer sous vide.

Poids : 1,5 g

e) Chloro-6 (chloro-4 phényl)-3 triazine-1,2,4

1,5 g du produit obtenu précédemment sont chauffés à reflux dans 30 ml d'oxychlorure de

phosphore pendant 4 h. Concentrer l'excès d'oxychlorure de phosphore sous vide, reprendre le résidu dans l'eau glacée, extraire au chlorure de méthylène, décanter, sécher sur sulfate de sodium, filtrer et concentrer sous vide.

Poids : 1,2 g

f) SR 42 833

1,2 g du produit obtenu précédemment sont chauffés à reflux dans 30 ml d'éthanol absolu en présence de 1,6 g d'hydroxy-4 pipéridine pendant 5 h.

Concentrer l'éthanol sous vide, reprendre le résidu dans l'eau, alcaliniser avec une solution de carbonate de sodium, extraire au chlorure de méthylène, décanter la phase organique, sécher sur sulfate de sodium, filtrer et concentrer sous vide. Recristalliser le résidu de l'éthanol absolu.

Poids : 0,75 g ; F. 188-190 °C

Exemple 20

(Chloro-3 phényl)-3 (hydroxy-4 pipéridino)-6 triazine-1,2,4 (SR 42 904)

On opère comme dans l'exemple 19, en remplaçant dans la première étape le chlorure de chloro-4 benzoyle par du chlorure de chloro-3 benzoyle.

On isole ainsi successivement :

— le N-(chloro-3 benzoyl)-aminoacétate d'éthyle : F. 70-72 °C (éther isopropylique)

— la (chloro-3 phényl)-3 tétrahydro-1,4,5,6 oxo-6 triazine-1,2,4 F. 228-230 °C (éthanol absolu)

— SR 42 904 ; F. 142-144 °C (acétate d'éthyle)

Les composés selon l'invention ont été étudiés en ce qui concerne leurs propriétés pharmacologiques. En particulier, ils ont été soumis aux épreuves suivantes :

1. Evaluation de l'activité anticonvulsivante

L'effet anticonvulsivant des produits chez la souris a été évalué sur un modèle de convulsions provoquées par un choc électrique et, sur un modèle de convulsions induites, par un agent chimique : la bicuculline.

a) Antagonisme des convulsions induites par un choc électrique

Le test utilisé a été légèrement modifié par rapport à celui de Swinyard et al. [Journal of Pharmacology and Experimental Therapeutics 106, 319-330 (1952)] et Asami et al. [Arzneimittel Forschung 24 (10), 1563-1568 (1974)]. Le matériel était composé d'un générateur de chocs Racia muni de deux électrodes oculaires délivrant un courant de 12,5 V pendant 0,3 s. Les lots étaient constitués de 10 souris Charles Rivers CDI, de poids compris entre 20 et 24 g.

Les produits ont été administrés, par voie orale, 60 min avant l'électrochoc. Les animaux ne présentant pas l'extension tonique des membres postérieurs ont été considérés comme protégés de la crise convulsive.

(Voir tableau page 13)

| Produits | Dose efficace médiane (DE 50) (1) d'antagonisme du choc électrique (mg/kg, p.o.) |
|---|---|
| SR 42 903 | 28 (25 - 32) |
| SR 42 904 | 59 (42 - 82) |
| SR 42 524 | 24 (18 - 32) |
| SR 42 642 | 40 (33 - 49) |
| SR 42 825 | 16 ( 8 - 29) |
| SR 42 826 | 26 (19 - 35) |
| SR 42 505 | 42 (36 - 50) |
| SR 42 824 | 20 (16 - 26) |
| SR 43 139 | 43 (37 - 51) |
| SR 43 159 | 83 (63 - 108) |
| SR 43 516 | 91 (57 - 122) |
| SR 43 543 | 19 (12 - 43) |
| SR 43 537 | 2,5 (0,9 - 3,4) |
| SR 43 539 | 2,5 (1,8 - 3,6) |
| SR 43 651 | 43 (31 - 56) |

(1) La DE 50 a été calculée par la méthode des probits, l'intervalle de confiance entre parenthèses a été établi pour le niveau de probabilité $p \leq 0{,}05$.

b) Antagonisme des convulsions et de la mortalité provoquées par la bicuculline

Les lots étaient constitués de 10 souris Charles River CDI, de poids compris entre 20 et 22 g. Les produits ont été administrés par voie orale, 60 min avant la bicuculline (0,8 mg/kg, i.v.). L'apparition de convulsions toniques ainsi que la mortalité ont été notées pendant les 60 min qui ont suivi l'injection de la bicuculline.

| Produits | Dose efficace médiane (DE 50) (1) d'antagonisme de la bicuculline (mg/kg, p.o.) | |
|---|---|---|
| | Convulsions toniques | Mortalité |
| SR 42 524 | 71 (58 - 87) | 72 (56 - 94) |
| SR 42 825 | 3,1 (2,3 - 3,7) | 3,3 (2,5 - 4,3) |
| SR 42 826 | 3,2 (2 - 5) | 3,4 (2,3 - 5) |
| SR 42 505 | 52 (38 - 72) | 54 (40 - 73) |
| SR 42 824 | 9,4 (7,8 - 11,5) | 8 (6,1 - 10,5) |
| SR 43 139 | 46 (35 - 59) | 44 (34 - 57) |
| SR 43 159 | 70 (48 - 101) | 60 (47 - 77) |
| SR 43 543 | 4,3 (3,1 - 5,8) | 3,9 (0,6 - 5,8) |
| SR 43 537 | 3,3 (2,4 - 5,2) | 3,0 (2,1 - 4,5) |
| SR 43 539 | 1,1 (0,5 - 2,8) | 0,7 (0,3 - 1,4) |
| SR 43 651 | 17,2 (11 - 32) | 15,5 (10 - 27) |

(1) La DE 50 a été calculée par la méthode des probits, l'intervalle de confiance entre parenthèses a été établi pour un niveau de probabilité $p \leq 0{,}05$.

Après administration orale chez la souris, les produits selon l'invention manifestent des propriétés anticonvulsivantes, tant vis-à-vis du choc électrique que de la bicuculline.

2. Evaluation de l'activité analgésique des produits

L'évaluation de l'activité analgésique des produits selon l'invention a été effectuée grâce au test de Koster [Federation Proceeding 18, 41 (1959)].

Les produits ont été administrés par voie orale chez la souris 30 min avant l'injection i.p. de 0,25 ml d'acide acétique 0,1 N en solution dans la gomme arabique à 10 %. Les contorsions qui suivent l'administration d'acide entre la cinquième et la dixième minute et entre la quinzième et la vingtième minute ont été comptabilisées chez les animaux témoins et les animaux traités.

| Produits | Dose mg/kg, p.o. | Antagonisme des mouvements de contorsions (pour cent/ témoins) |
|---|---|---|
| SR 42 482 | 25 | - 50 % |
| SR 42 833 | 25 | - 31 % |
| SR 42 825 | 25 | - 57 % |
| SR 42 826 | 25 | - 42 % |
| SR 42 505 | 25 | - 36 % |
| SR 42 824 | 25 | - 80 % |

Les produits selon l'invention sont capables d'antagoniser les mouvements de contorsion provoqués par l'administration d'acide acétique chez la souris, cet effet est prédictif d'une activité analgésique.

3. Evaluation de l'activité antithrombotique

Les produits de la présente invention ont manifesté une activité antithrombotique chez l'animal. Par exemple, le SR 42 524 (200 mg/kg, p. o.) a protégé les souris de la mortalité consécutive à l'injection de collagène.

4. Détermination de la dose létale chez la souris après administration aiguë

Les produits ont été administrés par voie orale, à des lots de 5 souris femelles Charles River CDI, de poids compris entre 20 et 24 g. Ils ont été solubilisés dans HCl 0,1 N. La toxicité a été relevée pendant les 72 h qui ont suivi l'administration des produits.

(Voir tableau page 15)

| Produits | Pour cent de toxicité ou DL 50 | | |
|---|---|---|---|
| | 250 mg/kg p.o. | 500 mg/kg p.o. | 1000 mg/kg p.o. |
| SR 42 482 | 0 | 0 | 0 |
| SR 42 696 | 0 | 0 | - |
| SR 42 904 | 0 | 0 | |
| SR 42 833 | 0 | 0 | - |
| SR 42 524 | 0 | 0 | 0 |
| SR 42 642 | 0 | 0 | - |
| SR 42 825 | 20 | 20 | 80 |
| SR 42 826 | 0 | 20 | - |
| SR 42 505 | 0 | 0 | 80 |
| SR 42 824 | 0 | 0 | - |
| SR 42 921 | 0 | 0 | - |
| SR 42 922 | 0 | 0 | 0 |
| SR 43 139 | 0 | 0 | 0 |
| SR 43 159 | 0 | 0 | 0 |
| SR 43 516 | 0 | 0 | 0 |
| SR 43 543 | 0 | 0 | 20 |

Les résultats exprimés en pourcentage d'animaux qui meurent dans les 72 h consécutives à l'administration orale des produits sont notés dans le tableau précédent.

Les doses létales de ces dérivés sont donc considérablement plus élevées que leurs doses actives dans les tests pharmacologiques décrits dans les précédents paragraphes.

Les essais ainsi effectués montrent que les produits selon l'invention présentent des propriétés pharmacologiques intéressantes et une faible toxicité. Par suite, ils peuvent être utilisés en thérapeutique humaine, notamment pour le traitement des affections psychiques, neurologiques, neuromusculaires, cardiovasculaires et inflammatoires.

En particulier, les produits selon l'invention peuvent être utilisés pour le traitement de la douleur, des états anxieux, des insomnies, de l'épilepsie, des troubles de la coagulation sanguine ainsi que des maladies inflammatoires.

Ces produits peuvent être administrés par voie orale ou par voie injectable. Les compositions pharmaceutiques peuvent être solides ou liquides et se présenter, par exemple, sous forme de comprimés, gélules, granulés, suppositoires ou préparations injectables.

La posologie peut varier dans de larges proportions, en particulier, suivant le type et la gravité de l'affection à traiter et suivant le mode d'administration. Le plus souvent, chez l'adulte, par voie orale, elle est comprise entre 1 mg et 500 mg par jour, éventuellement répartie en plusieurs prises.

**Revendications** (pour les Etats contractants : BE, CH, DE, FR, GB, IT, LI, LU, NL, SE)

1. Composés à noyau hétérocyclique azoté de formule générale :

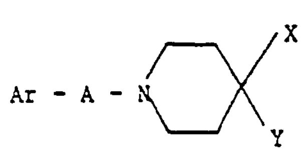 (I)

dans laquelle :

— Ar représente un groupe

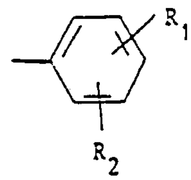

où

$R_1$ représente un atome d'hydrogène, un atome d'halogène, un groupe trifluorométhyle, un groupe alkyle inférieur (1 à 4 atomes de carbone), un groupe alcoxy inférieur (1 à 4 atomes de carbone), un groupe nitro, un groupe cyano ou un groupe hydroxyle ;

$R_2$ représente H ou un atome d'halogène, ou encore

— Ar représente un groupe naphtyle éventuellement substitué par un atome d'halogène ;

— A représente l'un des noyaux hétérocycliques azotés suivants :

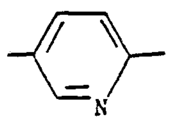 , 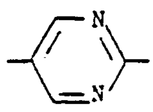 , 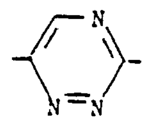 , 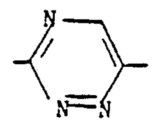

— X et Y, considérés isolément, désignent, l'un, un atome d'hydrogène, et, l'autre, un groupe $—OR_3$ dans lequel $R_3$ représente :

un atome d'hydrogène ;

un groupe

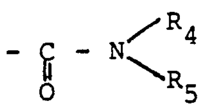

dans lequel $R_4$ représente l'hydrogène ou un groupe alkyle inférieur (1 à 4 atomes de carbone) et $R_5$ représente un groupe alkyle inférieur (1 à 4 atomes de carbone) ou encore

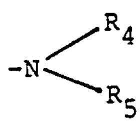

représente un groupe hydroxy-4 pipéridino ;

un groupe

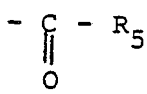

dans lequel $R_5$ est tel que défini ci-dessus ;

ou bien X et Y, considérés ensemble, constituent un groupe oxo (= 0), et les sels d'addition desdits composés avec des acides minéraux ou organiques.

2. Procédé pour l'obtention des composés selon la revendication 1, caractérisé en ce que l'on fait réagir le dérivé halogéné de formule Ar—A—Hal dans laquelle Ar et A sont tels que définis dans la revendication 1 et Hal est un atome d'halogène, avec une amine de formule

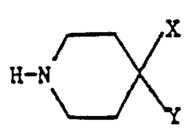

dans laquelle X et Y, considérés isolément, désignent, l'un, un atome d'hydrogène et l'autre, un groupe hydroxyle ou X et Y, pris ensemble, représentent un groupe oxo, éventuellement bloqué sous forme d'acétal, un excès, dans un solvant en chauffant à une température comprise entre 75 et 120 °C, en ce que, éventuellement, on hydrolyse le groupe acétal cyclique en milieu acide, en ce que, éventuellement, on transforme le groupe hydroxyle en ester ou en carbamate selon des procédés connus et en ce que, éventuellement, on transforme le composé obtenu en l'un de ses sels par action d'un acide minéral ou organique.

3. Composition pharmaceutique, caractérisée en ce qu'elle contient au moins un composé selon la revendication 1, en combinaison avec un véhicule pharmaceutiquement acceptable.

4. Composition pharmaceutique, active sur le système nerveux central, caractérisée en ce qu'elle contient au moins un composé selon la revendication 1, en combinaison avec un véhicule pharmaceutiquement acceptable.

5. Composition selon la revendication 4, caractérisée en ce qu'elle est sous une forme adaptée pour l'administration orale et en ce qu'elle contient unitairement de 1 à 500 mg dudit composé selon la revendication 1.

**Revendications** (pour l'Etat contractant AT)

1. Procédé de préparation de composés à noyau hétérocyclique azoté de formule générale :

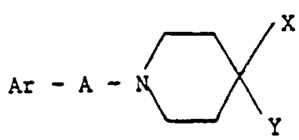

(I)

dans laquelle

— Ar représente un groupe

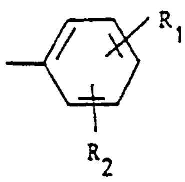

où

$R_1$ représente un atome d'hydrogène, un atome d'halogène, un groupe trifluorométhyle, un groupe alkyle inférieur (1 à 4 atomes de carbone), un groupe alcoxy inférieur (1 à 4 atomes de carbone), un groupe nitro, un groupe cyano ou un groupe hydroxyle ;

$R_2$ représente H ou un atome d'halogène, ou encore

— Ar représente un groupe naphtyle éventuellement substitué par un atome d'halogène ;

— A représente l'un des noyaux hétérocycliques azotés suivants :

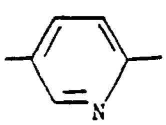

,

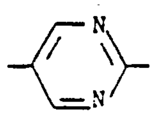

,

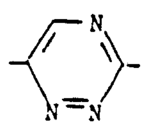

,

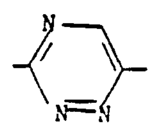

— X et Y, considérés isolément, désignent, l'un, un atome d'hydrogène et, l'autre, un groupe $—OR_3$ dans lequel $R_3$ représente :

un atome d'hydrogène ;

un groupe

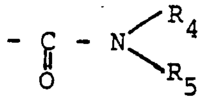

dans lequel $R_4$ représente l'hydrogène ou un groupe alkyle inférieur (1 à 4 atomes de carbone) et $R_5$ représente un groupe alkyle inférieur (1 à 4 atomes de carbone) ou encore

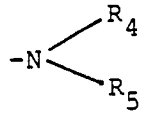

représente un groupe hydroxy-4 pipéridino ;

un groupe

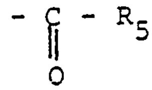

dans lequel $R_5$ est tel que défini ci-dessus ;
ou bien X et Y, considérés ensemble, constituent un groupe oxo (= 0), et de leurs sels d'addition desdits composés avec des acides minéraux ou organiques, caractérisé en ce qu'il consiste à faire réagir le dérivé halogéné de formule Ar—A—Hal dans laquelle Ar et A sont tels que définis ci-dessus et Hal est un atome d'halogène, avec une amine de formule

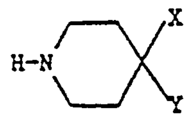

dans laquelle X et Y, considérés isolément, désignent, l'un, un atome d'hydrogène et l'autre, un groupe hydroxyle ou X et Y, pris ensemble, représentent un groupe oxo, éventuellement bloqué sous forme d'acétal, en excès, dans un solvant en chauffant à une température comprise entre 75 et 120 °C, à éventuellement hydrolyser le groupe acétal cyclique en milieu acide, à éventuellement transformer le groupe hydroxyle en ester ou en carbamate selon des procédés connus et à éventuellement transformer le composé obtenu en l'un de ses sels par action d'un acide minéral ou organique.

2. Utilisation des composés à noyau hétérocyclique de formule (I) préparés selon la revendication 1, en combinaison avec un véhicule pharmaceutiquement acceptable, pour la préparation de compositions pharmaceutiques.

3. Utilisation des composés à noyau hétérocyclique de formule (I) préparés selon la revendication 1, en combinaison avec un véhicule pharmaceutiquement acceptable, pour la préparation de compositions pharmaceutiques, actives sur le système nerveux central.

4. Utilisation selon la revendication 3, pour la préparation de compositions pharmaceutiques, sous une forme adaptée pour l'administration orale ; lesdites compositions contenant unitairement de 1 à 500 mg dudit composé de formule (I).

**Claims** (for Contracting States : BE, CH, DE, FR, GB, IT, LI, LU, NL, SE)

1. Compounds with a nitrogen-containing heterocyclic nucleus of general formula :

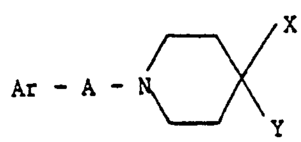

(I)

in which :

— Ar represents a group

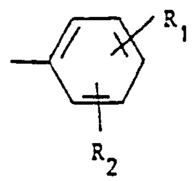

where

$R_1$ represents a hydrogen atom, a halogen atom, a trifluoromethyl group, a lower alkyl group (1 to 4 carbon atoms), a lower alkoxy group (1 to 4 carbon atoms), a nitro group, a cyano group or a hydroxyl group ;

$R_2$ represents H or a halogen atom ; where

— Ar represents a naphthyl group possibly substituted by a halogen atom ;

— A represents one of the following nitrogen-containing heterocyclic nuclei :

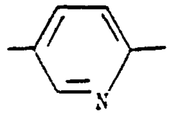 , 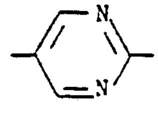 , 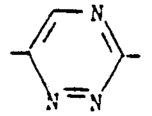 , 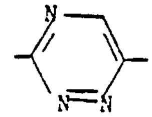

— X and Y, considered separately, designate, one, a hydrogen atom, and the other, an $—OR_3$ group in which $R_3$ represents :

a hydrogen atom ;

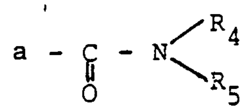

group in which $R_4$ represents hydrogen or a lower alkyl group (1 to 4 carbon atoms) and $R_5$ represents a lower alkyl group (1 to 4 carbon atoms)

or

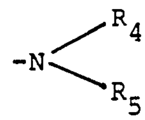

represents a 4-hydroxy piperidino group ;

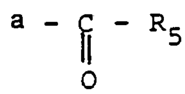

group in which $R_5$ is as defined hereinabove ;

or X and Y, considered together, constitute an oxo group (= 0), and the addition salts of said compounds with inorganic or organic acids.

2. Process for obtaining the compounds according to Claim 1, characterized in that the halogenated derivative of formula Ar—A—Hal in which Ar and A are as defined in Claim 1 and Hal is a halogen atom, is reacted with an amine of formula

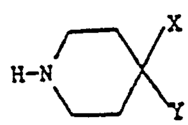

in which X and Y, considered separately, designate, one, a hydrogen atom and the other, a hydroxyl group or X and Y, taken together, represent an oxo group, possibly blocked in the form of acetal, in excess, in a solvent by heating to a temperature of between 75 and 120 °C, in that the cyclic acetal group is possibly hydrolyzed in acid medium, in that the hydroxyl group is possibly converted into ester or carbamate according to known processes, and in that the compound obtained is possibly converted into one of its salts by action of an inorganic or organic acid.

3. Pharmaceutical composition, characterized in that it contains at least one compound according to Claim 1, in combination with a pharmaceutically acceptable vehicle.

4. Pharmaceutical composition, active on the central nervous system, characterized in that it contains at least one compound according to Claim, in combination with a pharmaceutically acceptable vehicle.

5. Composition according to Claim 4, characterized in that it is in a form suitable for oral administration and in that it contains, per unit, from 1 to 500 mg of said compound according to Claim 1.

**Claims** (for Contracting State AT)

1. Process for preparing compounds with a nitrogen-containing heterocyclic nucleus of general formula :

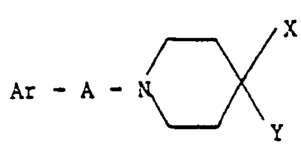

(I)

in which :

— Ar represents a group

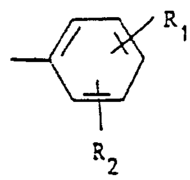

where

$R_1$ represents a hydrogen atom, a halogen atom, a trifluoromethyl group, a lower alkyl group (1 to 4 carbon atoms), a lower alkoxy group (1 to 4 carbon atoms), a nitro group, a cyano group or a hydroxyl group ;

$R_2$ represents H or a halogen atom

— Ar represents a naphthyl group possibly substituted by a halogen atom ;

— A represents one of the following nitrogen-containing heterocyclic nuclei :

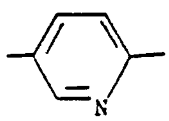 , 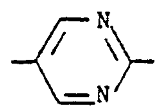 , 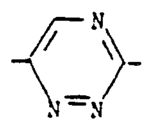 , 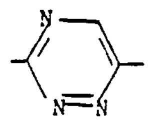

— X and Y, considered separately, designate, one, a hydrogen atom, and the other, an —$OR_3$ group in which $R_3$ represents :

a hydrogen atom ;

a 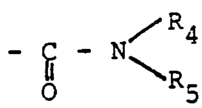

group in which $R_4$ represents hydrogen or a lower alkyl group (1 to 4 carbon atoms) and $R_5$ represents a lower alkyl group (1 to 4 carbon atoms)

or 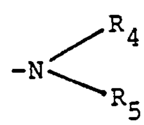

represents a 4-hydroxy piperidino group ;

a 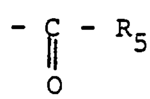

group in which $R_5$ is as defined hereinabove ;

or X and Y, considered together, constitute an oxo group (= 0), and their addition salts of said compounds with inorganic or organic acids,

characterized in that it consists in reacting the halogenated derivative of formula Ar—A—Hal, in which Ar and A are as defined hereinabove and Hal is a halogen, atom, with an amine of formula

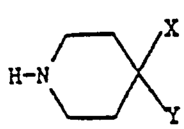

in which X and Y, considered separately, designate, one, a hydrogen atom and the other, a hydroxyl group or X and Y, taken together, represent an oxo group, possibly blocked in the form of acetal, in excess, in a solvent by heating to a temperature of between 75 and 120 °C, in possibly hydrolyzing the cyclic acetal group in acid medium, in possibly converting the hydroxyl group into ester or carbamate according to known processes, and in possibly converting the compound obtained into one of its salts by action of an inorganic or organic acid.

2. Use of the compounds with heterocyclic nucleus of formula (I) prepared according to Claim 1, in combination with a pharmaceutically acceptable vehicle, for the preparation of pharmaceutical compositions.

3. Use of the compounds with heterocyclic nucleus of formula (I) prepared according to Claim 1, in combination with a pharmaceutically acceptable vehicle, for the preparation of pharmaceutical compositions, active on the central nervous system.

4. Use according to Claim 3, for the preparation of pharmaceutical compositions, in a form suitable for oral administration ; said compositions containing, per unit, from 1 to 500 mg of said compound of formula (I).

**Patentansprüche** (für die Vertragsstaaten : BE, CH, DE, FR, GB, IT, LI, LU, NL, SE)

1. Verbindungen mit stickstoffhältigem Heterozyklus der allgemeinen Formel :

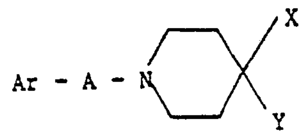

(I)

worin

— Ar ein Gruppe

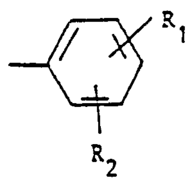

darstellt,
in welcher

$R_1$ für ein Wasserstoffatom, ein Halogenatom, eine Trifluormethylgruppe, eine Niedrigalkylgruppe (1 bis 4 Kohlenstoffatome), eine Niedrigalkoxygruppe (1 bis 4 Kohlenstoffatome), eine Nitrogruppe, eine Cyanogruppe oder eine Hydroxylgruppe steht ;

$R_2$ H oder ein Halogenatom darstellt ; oder aber

— Ar eine gegebenenfalls mit einem Halogenatom substituierte Naphthylgruppe darstellt ;

— A einen der folgenden stickstoffhältigen Hererozyklen darstellt :

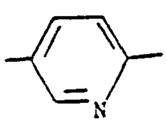 , 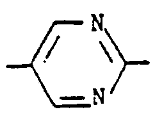 , 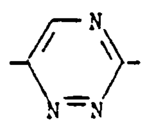 , 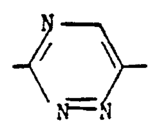

— X und Y, getrennt betrachtet, das eine eine Wasserstoffatom und das andere eine Gruppe —$OR_3$ bezeichnet, in welcher $R_3$ darstellt :

ein Wasserstoffatom ;

eine Gruppe

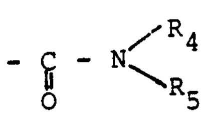

in der $R_4$ Wasserstoff oder eine Niedrigalkylgruppe (1 bis 4 Kohlenstoffatome) ist und $R_5$ eine Niedrigalkylgruppe (1 bis 4 Kohlenstoffatome) bezeichnet, oder aber

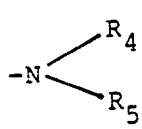

für eine 4-Hydroxypiperidinogruppe steht ;

eine Gruppe

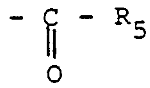

in der $R_5$ wie oben definiert ist; oder aber X und Y, zusammen betrachtet, eine Oxogruppe (= 0) bilden, und die Additionssalze dieser Verbindungen mit Mineral- oder organischen Säuren.

2. Verfahren zur Herstellung der Verbindungen nach Anspruch 1, dadurch gekennzeichnet, daß man das halogenierte Derivat der Formel Ar—A—Hal, worin Ar und A wie in Anspruch 1 definiert sind und Hal für ein Halogenatom steht, mit einem Amin der Formel

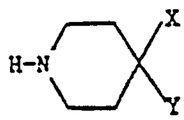

worin X und Y, getrennt betrachtet, das eine ein Wasserstoffatom und das andere eine Hydroxylgruppe darstellt, oder X und Y, zusammen genommen, eine gegebenenfalls in Acetalform blockierte Oxogruppe darstellen, im Überschuß in einem Lösungsmittel unter Erhitzen auf eine Temperatur zwischen 75 und 120 °C reagieren läßt, daß man gegebenenfalls die zyklische Azetalgruppe in saurem Milieu hydrolysiert, daß man gegebenenfalls die Hydroxylgruppe mittels bekannter Verfahren in den Ester oder in das Carbamat überführt, und daß man gegebenenfalls die erhaltene Verbindung durch Einwirken einer Mineral- oder organischen Säure in eines ihrer Salze überführt.

3. Pharmazeutische Zusammensetzung, dadurch gekennzeichnet, daß sie mindestens eine Verbindung nach Anspruch 1 in Kombination mit einem pharmazeutisch akzeptablen Vehikel enthält.

4. Pharmazeutische Zusammensetzung, die auf das Zentralnervensystem wirkt, dadurch gekennzeichnet, daß sie mindestens eine Verbindung nach Anspruch 1 in Kombination mit einem pharmazeutisch akzeptablen Vehikel enthält.

5. Zusammensetzung nach Anspruch 4, dadurch gekennzeichnet, daß sie in einer für orale Verabreichung aufbereiteten Form vorliegt und als Einheit 1 bis 500 mg der Verbindung nach Anspruch 1 enthält.

**Patentansprüche** (für den Verstragsstaat AT)

1. Verfahren zur Herstellung von Verbindungen mit stickstoffhältigem Heterozyklus der allgemeinen Formel:

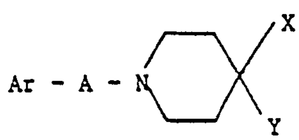

(I)

worin

— Ar eine Gruppe

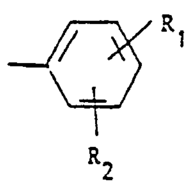

darstellt,
in welcher

$R_1$ für Wasserstoffatom, ein Halogenatom, eine Trifluormethylgruppe, eine Niedrigalkylgruppe (1 bis 4 Kohlenstoffatome), eine Niedrigalkoxygruppe (1 bis 4 Kohlenstoffatome), eine Nitrogruppe, eine Cyanogruppe oder eine Hydroxylgruppe steht;

$R_2$ H oder eine Halogenatom darstellt; oder aber

— Ar eine gegebenenfalls mit einem Halogenatom substituierte Naphthylgruppe darstellt;

— A einen der folgenden heterozyklisdchen Stickstoffkerne darstellt:

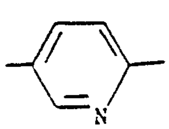 , 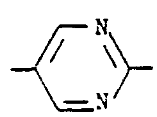 , 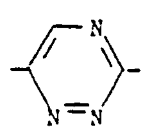 , 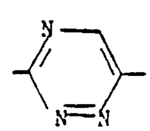

— X und Y, getrennt betrachtet, das eine ein Wasserstoffatom und das andere eine Gruppe —$OR_3$ bezeichnet, in welcher $R_3$ darstellt :

ein Wasserstoffatom ;

eine Gruppe

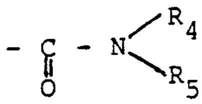

in der $R_4$ Wasserstoff oder eine Niedrigalkylgruppe (1 bis 4 Kohlenstoffatome) ist und $R_5$ eine Niedrigalkylgruppe (1 bis 4 Kohlenstoffatome) bezeichnet, oder aber

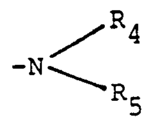

für eine 4-Hydroxypiperidinogruppe steht ;

eine Gruppe

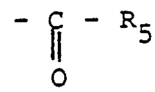

in der $R_5$ wie oben definiert ist ;

oder aber X und Y, zusammen betrachtet, eine Oxogruppe (=O) bilden,

und der Additionssalze dieser Verbindungen mit Mineral- oder organischen Säuren,

dadurch gekennzeichnet, daß es darin besteht, daß man das halogenierte Derivat der Formel Ar—A—Hal, worin Ar und A wie oben definiert sind und Hal für ein Halogenatom steht, mit einem Amin der Formel

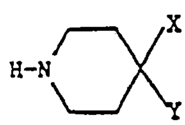

worin X und Y, gesondert betrachtet, das eine ein Wasserstoffatom und das andere ein Hydroxylgruppe darstellt, oder X und Y, zusammen genommen, eine gegebenenfalls in Acetalform blockierte Oxogruppe darstellen, im Überschuß in einem Lösungsmittel unter Erhitzen auf eine Temperatur zwischen 75 und 120 °C reagieren läßt, daß man gegebenenfalls die zyklische Azetalgruppe in saurem Milieu hydrolysiert, daß man gegebenenfalls die Hydroxylgruppe mittels bekannter Verfahren in den Ester oder in das Carbamat überführt, und daß man gegebenenfalls die erhaltene Verbindung durch Einwirken einer Mineral- oder organischen Säure in eines ihrer Salze überführt.

2. Verwendung der nach Anspruch 1 hergestellten Heterozyklus-Verbindungen der Formel (I) in Kombination mit einem pharmazeutisch akzeptablen Vehikel zur Herstellung von pharmazeutischen Zusammensetzungen.

3. Verwendung der nach Anspruch 1 hergestellten Heterozyklus-Verbindungen der Formel (I) in Kombination mit einem pharmazeutisch akzeptablen Vehikel zur Herstellung von pharmazeutischen Zusammensetzunge, die auf das Zentralnervensystem wirken.

4. Verwendung nach Anspruch 3 zur Herstellung von pharmazeutischen Zusammensetzungen in einer für orale Verabreichung aufbereiteten Form, welche Zusammensetzungen als Einheit 1 bis 500 mg der Verbindung der Formel (I) enthalten.